# EUROPEAN PATENT APPLICATION

(11) **EP 4 006 764 A1**
(43) Date of publication of application: **01.06.2022**
(21) Application number: 20845049.4
(22) Date of filing: 17.07.2020
(51) Int. Cl.: G06F 30/10, G06F 30/27

(54) **MATERIAL DESIGN SYSTEM, MATERIAL DESIGN METHOD, AND MATERIAL DESIGN PROGRAM**

(30) Priority: 23.07.2019 JP 2019135216
(71) Applicant: Showa Denko K.K., Tokyo 105-8518 (JP)
(72) Inventor: TAKEMOTO, Shimpei, Tokyo 105-8518 (JP); OKUNO, Katsuki, Tokyo 105-8518 (JP); MINAMI, Takuya, Tokyo 105-8518 (JP); AONUMA, Naoto, Tokyo 105-8518 (JP); TAKEDA, Eriko, Tokyo 105-8518 (JP); OKUNO, Yoshishige, Tokyo 105-8518 (JP)
(74) Representative: Strehl Schübel-Hopf & Partner
(86) International application number: PCT/JP2020/027909
(87) International publication number: WO 2021/015134

(57) **Abstract**

A material design system includes an expert terminal capable of using a model learning interface for performing machine learning of a model that inputs and outputs a correspondence between a design condition and a material property value of the material to be designed, and a plurality of general-purpose terminals configured to use a material design interface for estimating the material property value based on the design condition or estimating the design condition based on the material property value, by using a learned model that is created by the expert terminal and is for the material to be designed.

## Description

### [Technical Field]

The present disclosure relates to a material design system, material design method, and material design program.

### [Background Art]

Conventionally, when designing a material composed of a plurality of compositions or a material to be produced by combining a plurality of production conditions, an optimal solution capable of realizing desired material properties is acquired by repeating trial productions while adjusting material compositions and production conditions based on the experience of the material developer. However, in some cases, such an experience-based trial production by a material developer requires production repetition until the optimal design is acquired, which takes time and effort. Further, a condition search is often performed locally in the vicinity of a design condition that has been previously performed by the material developer, which is not suitable for a globally optimal design condition search.

Further, materials may be designed using simulation techniques such as first-principles calculation. In this case, forward problem analysis is performed to predict the material properties under the conditions set by the simulation engineer. However, even in the material design using simulation technology such as first-principles calculation, the results are output under the conditions set by the simulation engineer based on experience. Further, simulations usually are required to be executed for a long time until the result is obtained, which is not suitable for short time prediction or search for comprehensive material design.

Further, material designs using past experiment/evaluation databases and, recently, prediction of material properties (forward problem analysis) by applying machine learning to the database are performed. In order to facilitate machine learning, it is important to select appropriate learning methods, select objective variables, and adjust various hyperparameters for learning, and this setting may cause differences in learning results. In recent years, technology has been proposed to provide a platform for machine learning to users and to provide appropriate learning results more easily, regardless of proficiency of the users in machine learning, by the user performing an operation via the platform (see, for example, Patent Document 1).

### [Related Art Documents]

### [Patent Documents]

[Patent Document 1] Japanese Laid-open Patent Application Publication No. 2019-23906

### [Summary of Invention]

### [Problem to be Solved by Invention]

However, in the parameter setting of machine learning, even if the operation and the setting procedure are simplified, it is still considered quite difficult for a person unskilled in machine learning such as a material designer to perform this parameter setting. It still holds true that more appropriate settings can be achieved when machine learning is performed by an experienced person such as a data scientist who is familiar with statistics, machine learning, computer science, information science, or the like, and thus this is still advantageous. However, there are few data scientists in the field of material design, and machine learning experts are not necessarily around each material design system.

The purpose of the present disclosure is to provide a material design system, a material design method, and a material design program that can use a learning result of high-quality machine learning in a wide range by utilizing the know-how of a small number of machine learning experts.

### [Means for Solving Problem]

The present disclosure includes the following configurations.
(1) A material design system for designing a material to be designed including a material composed of a plurality of compositions or a material produced by combining a plurality of production conditions, the material design system comprising an expert terminal capable of using a model learning interface for performing machine learning of a model that inputs and outputs a correspondence between a design condition and a material property value of the material to be designed, and a plurality of general-purpose terminals configured to use a material design interface for estimating the material property value based on the design condition or estimating the design condition based on the material property value, by using a learned model that is created by the expert terminal and is for a specific material to be designed.
(2) The material design system according to the above-described Item (1) further comprising an intermediate device for storing the learned model created by the expert terminal, wherein the plurality of general-purpose terminals, by using the learned model stored in the intermediate device, estimate the material property value based on the design condition or estimate the design condition based on the material property value.
(3) The material design system according to the above-described Item (1) or (2), wherein communication between the model learning interface and the material design interface is performed via a network line.
(4) The material design system according to the above-described Item (1) or (2), wherein the model learning interface and the material design interface are installed in a cloud server, and communication between the model learning interface and the material design interface is performed by communication in the cloud server.
(5) The material design system according to the above-described Item (1) or (2), wherein the model learning interface and the material design interface are installed in separate software compatible with each other.
(6) The material design system according to the above-described Items (1) to (5), wherein the expert terminal includes a learning condition setting unit configured to set various conditions for machine learning of the model, a model learning unit configured to perform machine learning of the model based on the various conditions, and a model output unit configured to output the learned model.
(7) The material design system according to the above-described Items (1) to (6), wherein the general-purpose terminal includes a design condition setting unit configured to set a specified range of the design condition of the material to be designed, a comprehensive prediction point generation unit configured to generate a plurality of comprehensive prediction points within the specified range set by the design condition setting unit, a design condition-material property table for storing a data set associated with each point of the comprehensive prediction point, wherein the material property value is calculated by inputting the comprehensive prediction point generated by the comprehensive prediction point generation unit into the learned model, a required property setting unit configured to set a specified range of a required property of the material to be designed, and a design condition extraction unit configured to extract a data set satisfying the required property set by the required property setting unit from the design condition-material property table.
(8) The material design system according to the above-described Items (7), wherein the general-purpose terminal further includes a design condition adjustment unit configured to adjust a range of the design condition of the data set extracted by the design condition extracting unit, and the design condition extraction unit further narrows down the data set satisfying the design condition adjusted by the design condition adjustment unit from the extracted data set.
(9) A material design method of designing a material to be designed including a material composed of a plurality of compositions or a material produced by combining a plurality of production conditions, the material design method comprising performing machine learning of a model that inputs and outputs a correspondence between a design condition and a material property value of the material to be designed, and estimating the material property value based on the design condition or estimating the design condition based on the material property value, by using a learned model that is created by the expert terminal and is for the material to be designed.
(10) A material design program for designing a material to be designed including a material composed of a plurality of compositions or a material produced by combining a plurality of production conditions, the material design program causing a computer to implement as a learning function of performing machine learning of a model that inputs and outputs a correspondence between a design condition and a material property value of the material to be designed, and an estimation function of, by using a learned model that is created by the expert terminal and is for the material to be designed, estimating the material property value from the design condition or estimating the design condition from the material property value.

### [Advantageous Effects of Invention]

According to the present disclosure, a material design system, a material design method, and a material design program that can use a learning result of high-quality machine learning in a wide range by utilizing the know-how of a small number of machine learning experts can be provided.

### [Brief Description of Drawings]

FIG. 1 is a block diagram illustrating a schematic configuration of a material design system according to an embodiment; FIG. 2 is a block diagram illustrating a modification of a schematic configuration of the material design system according to the embodiment;
FIG. 3 is a diagram illustrating functional blocks of an expert terminal;
FIG. 4 is a diagram illustrating an example of an input screen of a learning condition setting unit in a model learning interface;
FIG. 5 is a diagram illustrating an example of the input screen of the learning condition setting unit in the model learning interface;
FIG. 6 is a diagram illustrating an example of the input screen of the learning condition setting unit in the model learning interface;
FIG. 7 is a diagram illustrating an example of the input screen of the learning condition setting unit in the model learning interface;
FIG. 8 is a diagram illustrating an example of the input screen of the learning condition setting unit in the model learning interface;
FIG. 9 is a diagram illustrating an example of the input screen of the learning condition setting unit in the model learning interface;
FIG. 10 is a diagram illustrating functional blocks of a general-purpose terminal;
FIG. 11 is a diagram illustrating an example of an input screen of a design condition setting unit in a material design interface;
FIG. 12 is a diagram illustrating an example of an output screen of a forward problem analysis unit in the material design interface;
FIG. 13 is a diagram illustrating an example of an input screen of a required property setting unit;
FIG. 14 is a diagram illustrating an example of an output screen of a reverse problem analysis unit;
FIG. 15 is a diagram illustrating an output screen which is another example of the output screen of the reverse problem analysis unit;
FIG. 16 is a diagram illustrating a hardware configuration of the expert terminal and the general-purpose terminal as blocks;
FIG. 17 is a flowchart of a model learning processing performed by the expert terminal;
FIG. 18 is a flowchart of a forward problem analysis processing performed by the forward problem analysis unit of the general-purpose terminal; and
FIG. 19 is a flowchart of a reverse problem analysis processing performed by the reverse problem analysis unit and a design condition adjustment unit of the general-purpose terminal.

### [Description of Embodiments]

Hereinafter, embodiments will be described with reference to the drawings. In order to facilitate understanding of the description, the same components are indicated by the same reference numerals as possible in the drawings, and duplicate description is omitted.

### <Overall configuration of material design system>

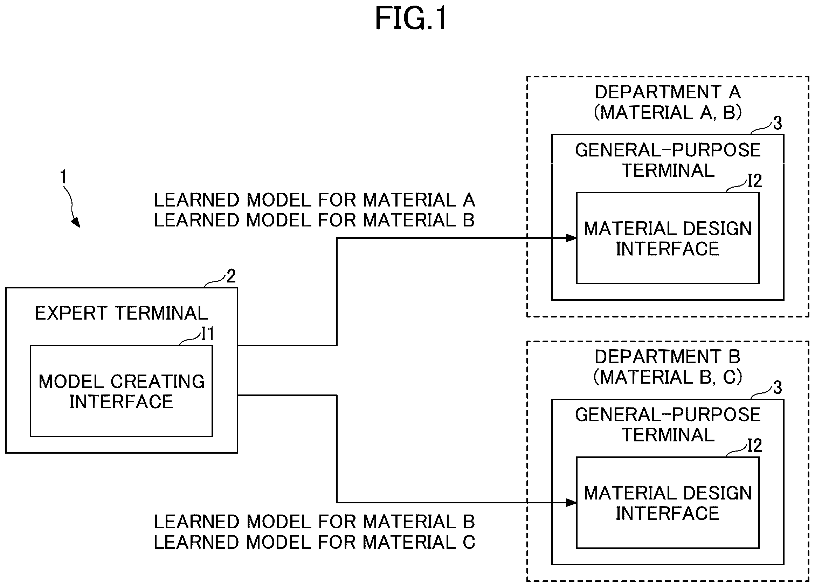

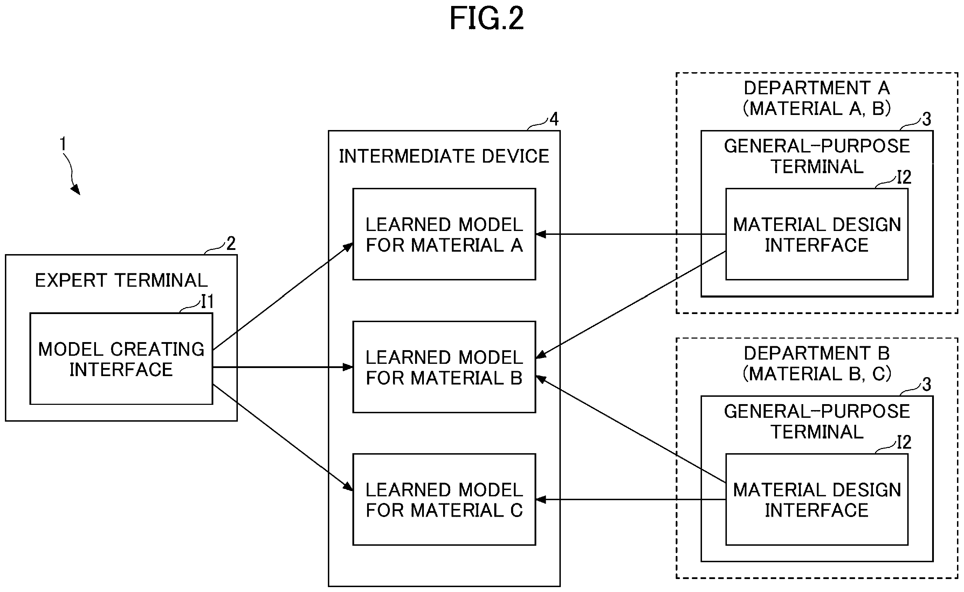

The configuration of a material design system 1 according to an embodiment will be described with reference to FIG. 1 and FIG. 2. FIG. 1 is a block diagram illustrating a schematic configuration of the material design system 1 according to the embodiment. The material design system 1 is an apparatus for designing a material to be designed including a material composed of a plurality of compositions or a material produced by combining a plurality of production conditions.

The material design system 1 can be applied to a design of organic materials such as synthetic rubber, synthetic resin and synthetic elastomer, metal materials such as alloys and steel, and materials in general such as inorganic materials and composite materials. In short, the material to be designed of the material design system 1 includes materials composed of a plurality of compositions, or materials produced by combining a plurality of production conditions/treatments (such as temperature, pressure, processing, oxidation treatment, acid treatment, proportion, mixture, and stirring).

As illustrated in FIG. 1, the material design system 1 includes a single expert terminal 2 and a plurality of (two in FIG. 1) general-purpose terminals 3.

The expert terminal 2 is a device capable of using a model learning interface I1. The model learning interface I1 is an interface for performing machine learning of a model that inputs/outputs a correspondence between the design condition and the material property value of the material to be designed. The model learning interface I1 is a Graphical User Interface (GUI) or an Application Programming Interface (API) for using programming by commands. The model learning interface I1 of the expert terminal 2 is used by a machine learning expert such as a data scientist.

The general-purpose terminal 3 is a device capable of using a material design interface I2 for a specific material to be designed. The material design interface I2 is an interface for, by using a learned model for the specific material to be designed created by the expert terminal 2, estimating a material property value from the design condition or estimating a design condition from the material property value. The material design interface I2 is a GUI with high user operability. The material design interface I2 of the general-purpose terminal 3 is used by a person unskilled in machine learning such as a material designer, that is, a non-data scientist.

For example, when the material design system 1 is provided in one company, the general-purpose terminal 3 is installed in each of the multiple design departments in the company.
At this time, the general-purpose terminal 3 is not required to be installed in all departments. In the example of FIG. 1, two departments, department A and department B, are exemplified, and in the department A, materials A and B are designed as materials to be designed. In the department B, the case where materials B and C are designed as the materials to be designed is exemplified.

In the expert terminal 2, a learned model is created by using the model learning interface I1. The expert terminal 2 and the general-purpose terminal 3 are communicably connected, and the learned model is transmitted from the expert terminal 2 to each general-purpose terminal 3. In the case of the present example, the expert terminal 2 creates three types of learned models used for the materials A, B, and C of the material to be designed, respectively. The learned models are transmitted to the general-purpose terminal 3 of each department according to the material handled in each department. In the example of FIG. 1, a learned model for the material A and a learned model for the material B are transmitted to the general-purpose terminal 3 of the department A that handles the materials A and B, and a learned model for the material B and a learned model for the material C are transmitted to the general-purpose terminal 3 of the department B that handles the materials B and C.

In the material design system 1, the model learning interface I1 and the material design interface 12, which can operate independently, can be linked by a network connection or the like. The material design interface I2 is configured such that a material developer who is not a data scientist can design a material without performing machine learning. Further, the material design interface I2 has a highly versatile design regardless of the type of material. The material design interface I2 is configured to be applicable to the development of all kinds of materials by providing information by file transfer or the like from the model learning interface I1.

As illustrated in FIG. 1, in the material design system 1, the number of expert terminals 2 capable of using the model learning interface I1 is smaller than that of the general-purpose terminals 3 capable of using the material design interface 12, and each of the expert terminals 2 and 3 is a separate device. This enables the material design system 1 according to the present embodiment to share the learned model created by the expert terminal 2 with a large number of general-purpose terminals 3. Therefore, a learning result of high-quality machine learning can be used in the multiple material design departments by utilizing the know-how of a small number of machine learning experts. That is, it is possible to obtain the effect that the learning result of high-quality machine learning in a wide range can be used.

In general, because a lot of labor, costs, and time are required to master machine learning as well as the fields of statistics, computer science, and information science on which machine learning is based, advanced machine learning is difficult for a material developer who is not an expert in data analysis (who is not a data scientist). Further, the lack of data scientists is being criticized in the world, making it difficult to secure sufficient personnel within the organization of material manufacturers. In response to such a problem, in the material design system 1 of the present embodiment, as described above, a system that efficiently supports material design to a large number of material development departments inside or outside the organization of a material manufacturer can be provided by setting up a small number of expert terminals 2 with respect to the general-purpose terminal 3, on the basis of securing a few data scientists.

In the present embodiment, the material design system 1 is provided in one company, and the general-purpose terminals 3 are provided in each of the multiple design departments in the one company, however, providing a method of the material design system 1 is not limited to this. For example, one material design system 1 may be provided across multiple companies or organizations, and a general-purpose terminal 3 may be installed for each company or organization. Alternatively, a material design system 1 may be provided in one group, and multiple general-purpose terminals 3 may be provided in the group according to the purpose.

FIG. 2 is a block diagram illustrating a modification of a schematic configuration of the material design system 1 according to the embodiment. As illustrated in FIG. 2, a material design system 1 may be configured to include an intermediate device 4 for storing a learned model created by an expert terminal 2. In the configuration of FIG. 2, multiple general-purpose terminals 3 use the learned model stored in the intermediate device 4 to estimate a material property value from the design condition or a design condition from the material property value.

By providing the intermediate device 4 in this way, each general-purpose terminal 3 can access and use the learned model stored in the intermediate device 4. Therefore, an operation such as individually distributing the data of the learned model to each terminal in order for each general-purpose terminal 3 to use the learned model becomes non-mandatory. Accordingly, machine learning can be performed more easily using the learned model. Further, because the availability of each general-purpose terminal 3 can be set by the access privilege to each learned model of the intermediate device 4, an allocation of the learned models that can be used by each general-purpose terminal 3 can be easily managed.

In this regard, communication between the expert terminal 2 and the general-purpose terminal 3, that is, communication between the model learning interface I1 and the material design interface I2 can be implemented, for example, in the following three types. By corresponding to various types of communication in such a way, the variation in implementation of the material design system 1 can be increased and the versatility can be improved.

(Type 1: Network system) Communication between the model learning interface I1 and the material design interface I2 is performed via a network line.

The server on which the model learning interface I1 is installed (expert terminal 2) and the server on which the material design interface I2 is installed (general-purpose terminal 3) are connected by a network. Information such as a learned model and a names file (material name, material property name, design condition item name) can be transferred from the server of the model learning interface I1 to the server of the material design interface 12. The learned model is a format such as a pickle file or a joblib file, and the names file is a format such as a text file, a CSV file, a JSON file, or an XML file.

The method of providing the above-mentioned information is not limited to the file transfer, and may be a format in which information stored in a recording medium such as a semiconductor memory (for example, a flash memory) or a disk medium (for example, a DVD-ROM) is transferred.

The information transfer from the model learning interface I1 to the material design interface 12, as illustrated in FIG. 1, may be performed directly from the server of the model learning interface I1 to the server of the material design interface 12, or, as illustrated in FIG. 2, after transferring the information from the model learning interface I1 to the intermediate server or the directory (intermediate device 4), the material design interface I2 may be allowed to access to the intermediate device 4.

### (Type 2: Web service)

The model learning interface I1 and the material design interface I2 are installed on the cloud server, and the communication between the model learning interface I1 and the material design interface I2 is performed by communication in the cloud server.

The model learning interface I1 and the material design interface I2 are separate interfaces installed in a cloud server such as Amazon Web Services (AWS, registered trademark) and Google Cloud Platform (GCP, registered trademark;), and communication within the cloud server is secured. In the cloud server, information related to the learned model and the name is transmitted by a file transfer or is transmitted by a transfer of information stored in a recording medium such as a semiconductor memory (for example, a flash memory) or a disk medium (for example, a DVD-ROM) is transferred, from the instance of the model learning interface I1 to the instance of the material design interface 12.

The information transfer from the model learning interface I1 to the material design interface 12, as illustrated in FIG. 1, may be performed directly from the instance of the model learning interface I1 to the instance of the material design interface 12, or, as illustrated in FIG. 2, the material design interface may be allowed to access to the intermediate device after the transferring of the information from model learning interface I1 to the intermediate instance or the directory (intermediate device 4).

### (Type 3: Software)

The model learning interface I1 and the material design interface I2 are installed in separate software compatible with each other.

The software of the model learning interface I1 can output the learned model and the names file to the outside, and the software of the material design interface I2 can read the learned model and the names file.

In the examples of FIG. 1 and FIG. 2, although the configuration in which the expert terminal 2 capable of using the interface I1 for model learning and the general-purpose terminal 3 capable of using the interface I2 for material design are separate devices is illustrated, the expert terminal 2 and at least one of the general-purpose terminal 3 may be the same device. If a large number of material design interfaces I2 can be used with respect to the model learning interface I1 even in a single device, a learning result of high-quality machine learning can be used in the multiple material design departments by utilizing the know-how of a small number of machine learning experts. That is, it is possible to obtain the effect that the learning result of high-quality machine learning in a wide range can be used.

### <Explanation of expert terminal functions>

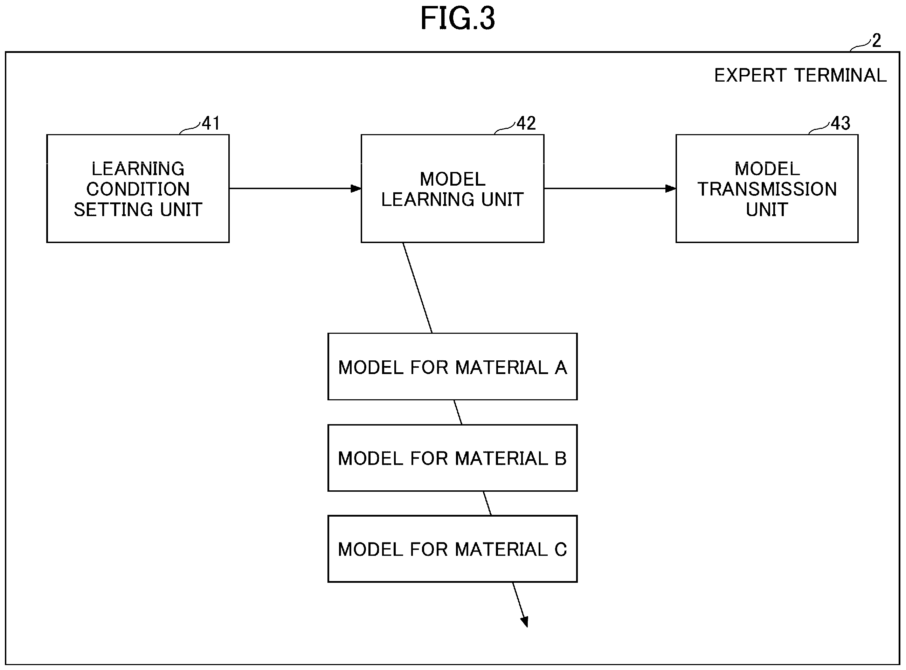

The functional configuration of the expert terminal 2 will be described with reference to FIG. 3 to FIG. 9. FIG. 3 is a diagram illustrating functional blocks of the expert terminal 2.

As illustrated in FIG. 3, the expert terminal 2 includes a learning condition setting unit 41, a model learning unit 42, and a model transmission unit (model output unit) 43.

The learning condition setting unit 41 sets various conditions for machine learning of the model.

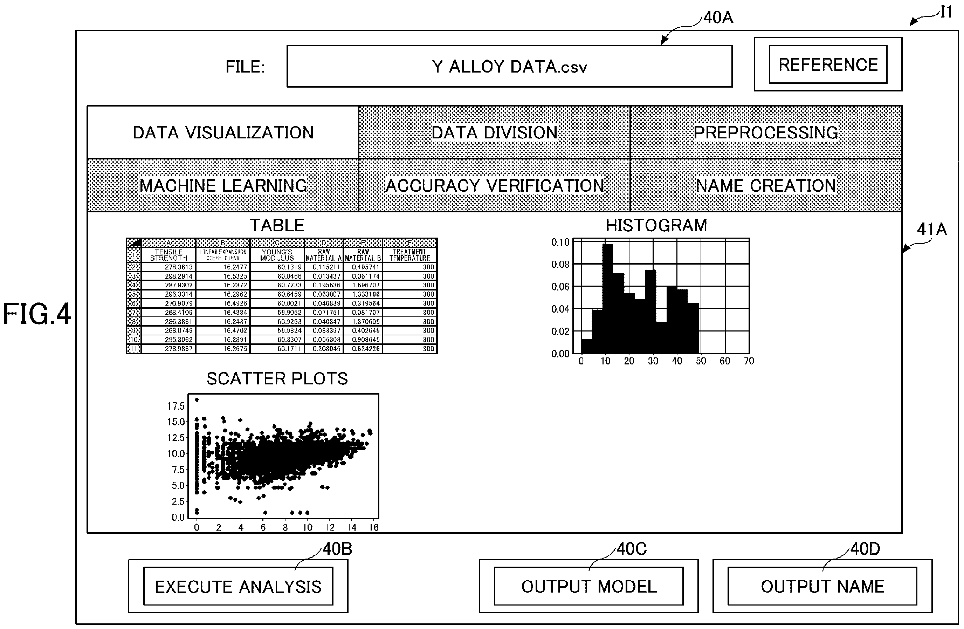

FIG. 4 to FIG. 9 are diagrams illustrating examples of input screens 41A to 41F of the learning condition setting unit 41 in the model learning interface.

As illustrated in FIG. 4, the input screen 41A is displayed as a tabbed window of "data visualization." The contents of the data file for model learning are displayed in various ways on the input screen 41A. The model learning interface I1 includes a function of reading a data file such as a CSV format, and in FIG. 4, the data file "Y alloy data.csv" is selected and read in the file selection box 40A. In this data file, a large number of sets of model input (design conditions) and model output (material property values) data sets related to Y alloy are recorded. On the input screen 41A of FIG. 4, the data set included in the read data file is displayed as a table, and the numerical values included in the data set are illustrated in histograms and scatter plots.

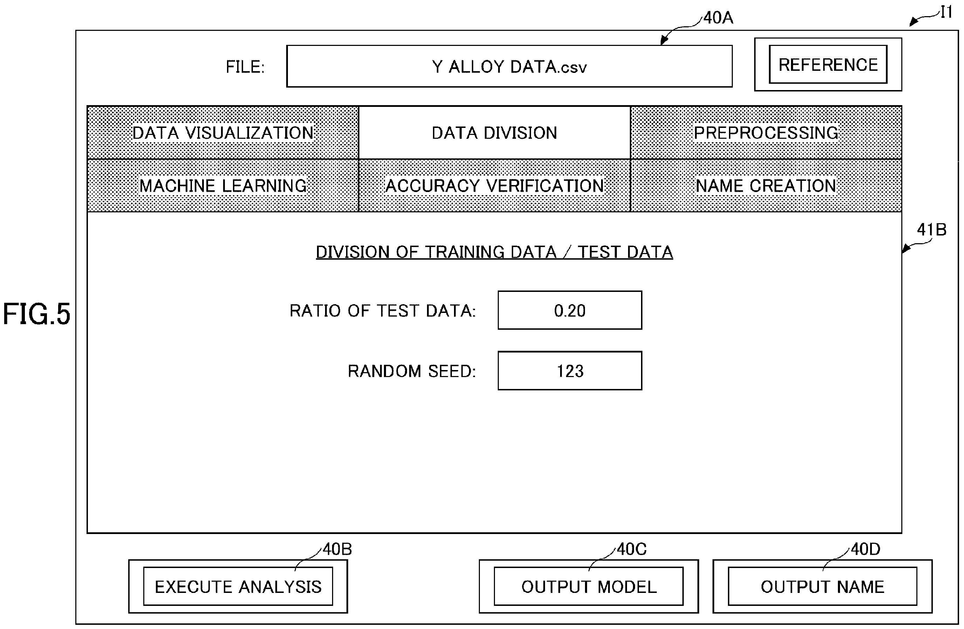

As illustrated in FIG. 5, an input screen 41B is displayed as a tabbed window of "data division." On the input screen 41B, the data set included in the read data file can be input as numerical values of various conditions (ratio of test data, random seed, etc.) of how to divide into training data and test data.

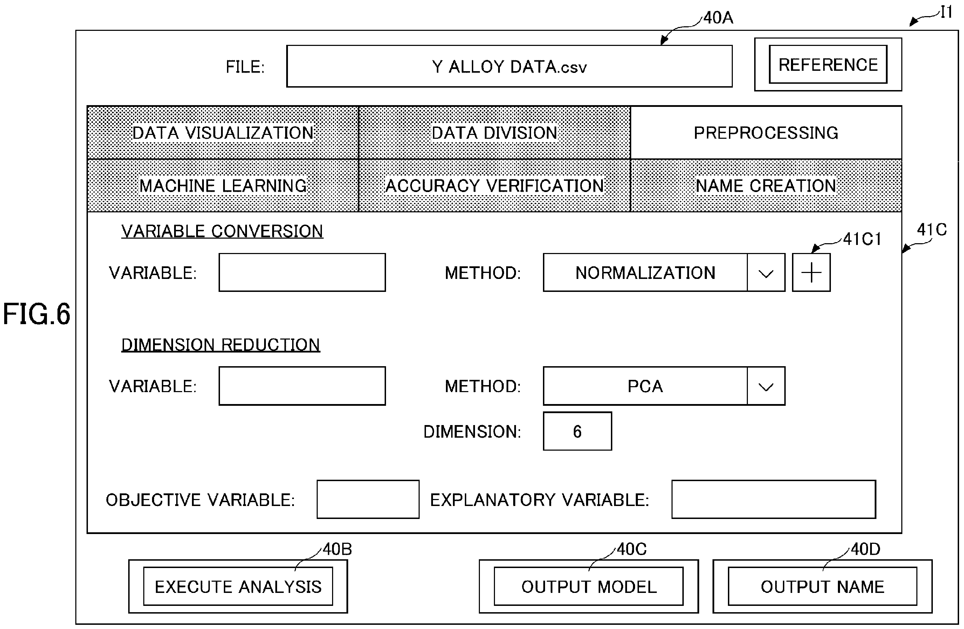

As illustrated in FIG. 6, an input screen 41C is displayed as a tabbed window of "preprocessing." On the input screen 41C, settings for converting and normalizing variables included in the data set using such as a function can be input. Also, settings for reducing the dimensions of variables included in the data set using, for example, principal component analysis (PCA), settings for assigning variables included in the data set to the objective variable and explanatory variable of the model, and the like can be input.

In the screen example of FIG. 6, a "+" button 41C1 is displayed in the item field of variable transformation, and for example, by pressing the "+" button 41C1, an entry field regarding a method and the like can be added.

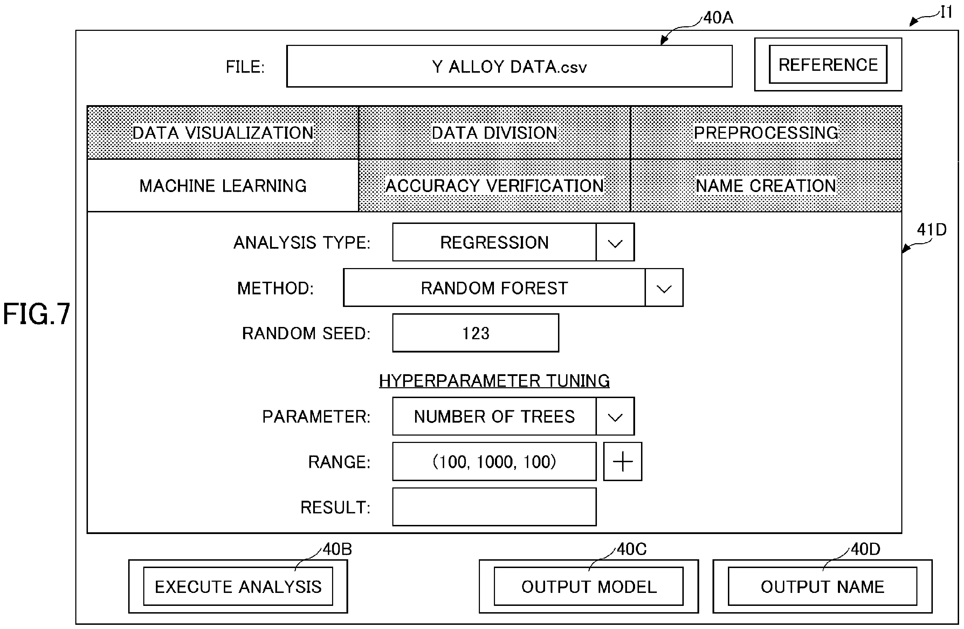

As illustrated in FIG. 7, an input screen 41D is displayed as a tabbed window of "machine learning." On the input screen 41D, selection of the machine learning method, settings of hyperparameter tuning and the like can be input. In the screen example of FIG. 7, "(100, 1000, 100)" is entered as the range of the hyper tuning parameter, and these numbers indicate the range of hyper tuning parameters (minimum, maximum, step size).

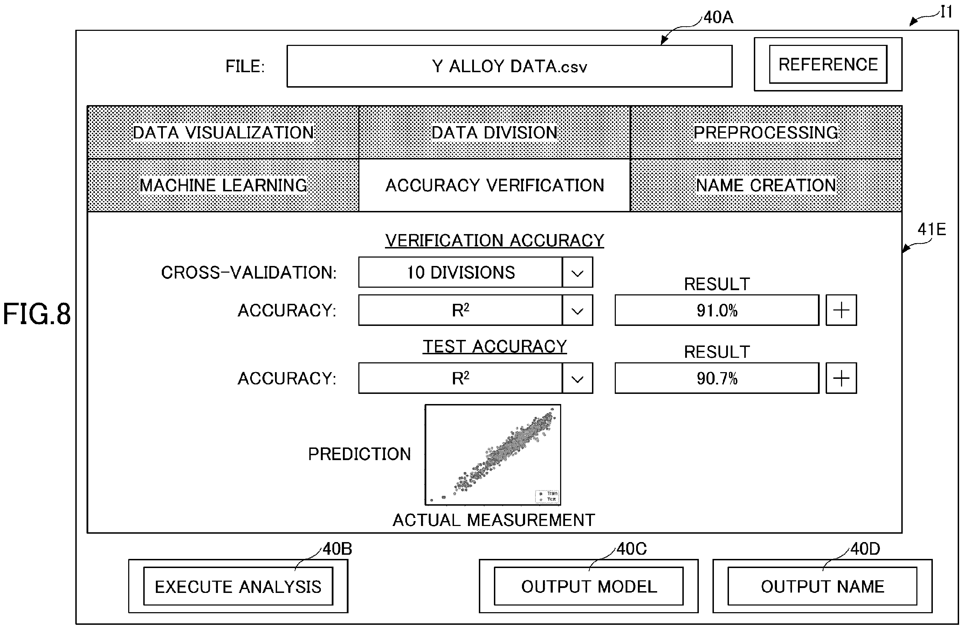

As illustrated in FIG. 8, an input screen 41E is displayed as a tabbed window for "accuracy verification." On the input screen 41E, settings of a function to evaluate a prediction accuracy of the learned model by cross-validation, settings of a function to evaluate the prediction accuracy of the learned model using test data, and settings of a function to illustrate a prediction result by the learned model can be input.

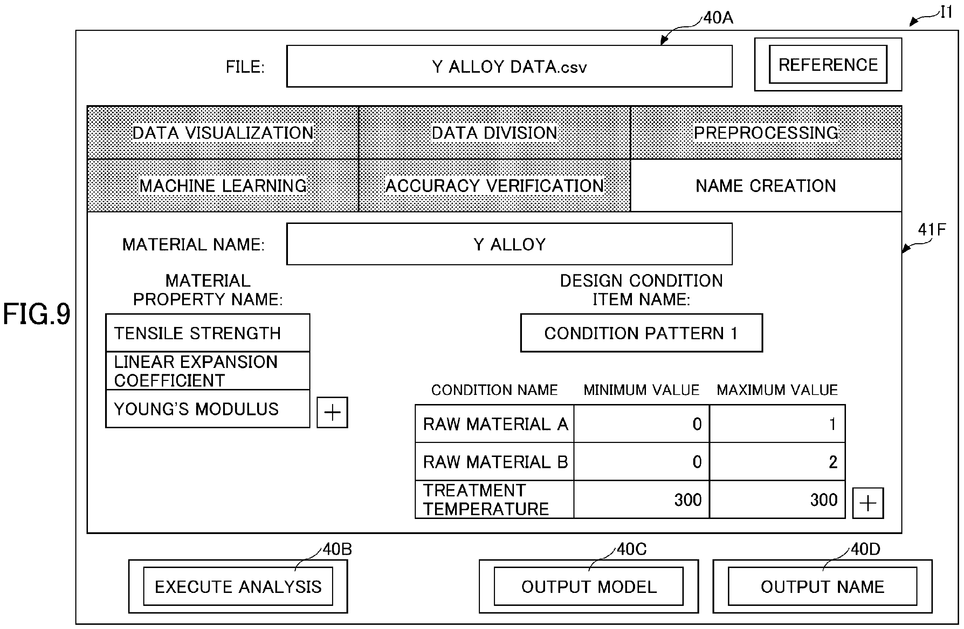

As illustrated in FIG. 9, an input screen 41F is displayed as a tabbed window for "name creation." On the input screen 41F, the learned model is output to the outside after designating the model name and the material name. Further, on the input screen 41F, the material name, the material property name, and the design condition item name can be input, and settings for output these to the outside in the format of the text file, the CSV file, the JSON file, the XML file, or the like can be input. Each name of the material property name and the design condition item name can be manually input, or the name described in the read data file may be automatically reflected. A material property name file and a design condition item name file are associated with the material name. In the material property name file and the design condition item name file, only each name of the material property name and the design condition item name may be input, or the default value in the range of possible values may be input.

The input screens 41A to 41F may be switched by tabs as illustrated in FIG. 4 to FIG. 9, and an analysis flow may be configured using icons corresponding to the functions provided in the input screens 41A to 41F and the arrows connecting the icons to set learning conditions according to the analysis flow.

Returning to FIG. 3, the model learning unit 42 performs machine learning of the model based on various conditions set by the learning condition setting unit 41. By pressing the "analysis execution" button 40B of the model learning interface I1, machine learning of the model is performed under the learning conditions set in FIG. 4 to FIG. 8. In the examples of FIG. 1 to FIG. 3, machine learning is performed on the "model for material A", "model for material B", and "model for material C" assigned to the materials A, B, and C of the material to be designed, respectively, to create learned models individually using the design of the materials A, B, and C.

The model learning unit 42 can perform machine learning such as regression and classification. As a method, generalized linear (Lasso, Ridge, Elastic Net, Logistic), Kernel Ridge, Bayesian Ridge, Gaussian Process, k-Nearest Neighbor, Decision Tree, Random Forest, AdaBoost, Bagging, Gradient Boosting, Support Vector Machine, Neural Network, Deep Learning, and the like may be used.

The model transmission unit 43 outputs the learned model. After designating the model name and the material name, the learned model is output to the outside by pressing the " output model" button 40C of the model learning interface I1. Further, by pressing the "output name" button 40D, a names file including various names (material name, material property name, design condition item name) set in FIG. 9 is output to the outside.

In this way, the expert terminal 2 includes the learning condition setting unit 41 that sets various conditions for machine learning of the model, the model learning unit 42 that performs machine learning of the model based on various conditions, and the model transmission unit 43 that outputs the learned model 13. This enables fine adjustment of various conditions of machine learning of the model, so that machine learning suitable for various purposes can be performed. Further, the expert terminal 2 is particularly effective when the expert terminal 2 is used by a machine learning expert such as a data scientist, because more appropriate condition settings can be expected based on the knowledge of the expert.

### <Functional explanation of general-purpose terminal>

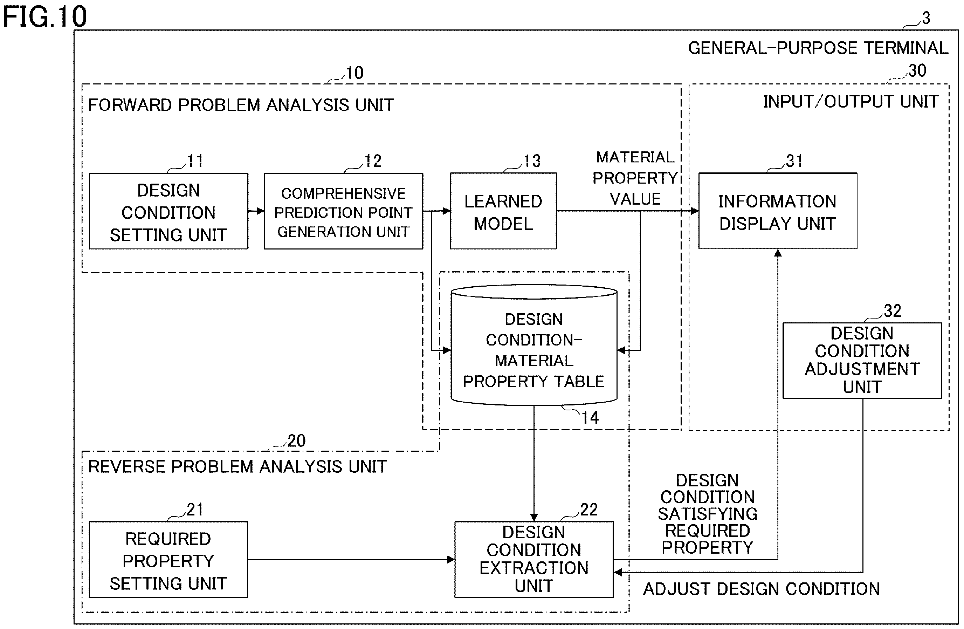

The functional configuration of the general-purpose terminal 3 will be described with reference to FIG. 10 to FIG. 15. FIG. 10 is a diagram illustrating functional blocks of the general-purpose terminal 3.

As illustrated in FIG. 4, the general-purpose terminal 3 includes a forward problem analysis unit 10, a reverse problem analysis unit 20, and an input/output unit 30.
The forward problem analysis unit 10 uses the learned model 13 to output a material property that satisfies the design condition desired by the material designer. The reverse problem analysis unit 20 outputs the design condition satisfying the desired property required by the material designer by using a design condition-material property table 14 created based on the output result of the forward problem analysis unit 10. The input/output unit 30 displays the output results of the forward problem analysis unit 10 and the reverse problem analysis unit 20 to present to the material designer, and accepts the adjustment operation of the output results by the material designer.

The forward problem analysis unit 10 includes a design condition setting unit 11, a comprehensive prediction point generation unit 12, the learned model 13, and the design condition-material property table 14.

The design condition setting unit 11 is configured to set a specified range of the design condition of the material to be designed. The design condition setting unit 11 can set the specified range of the design condition of the material to be designed by, for example, displaying a design condition input screen on the material design interface I2 to prompt the material designer to input the type of the material to be designed and the specified range of the design condition.

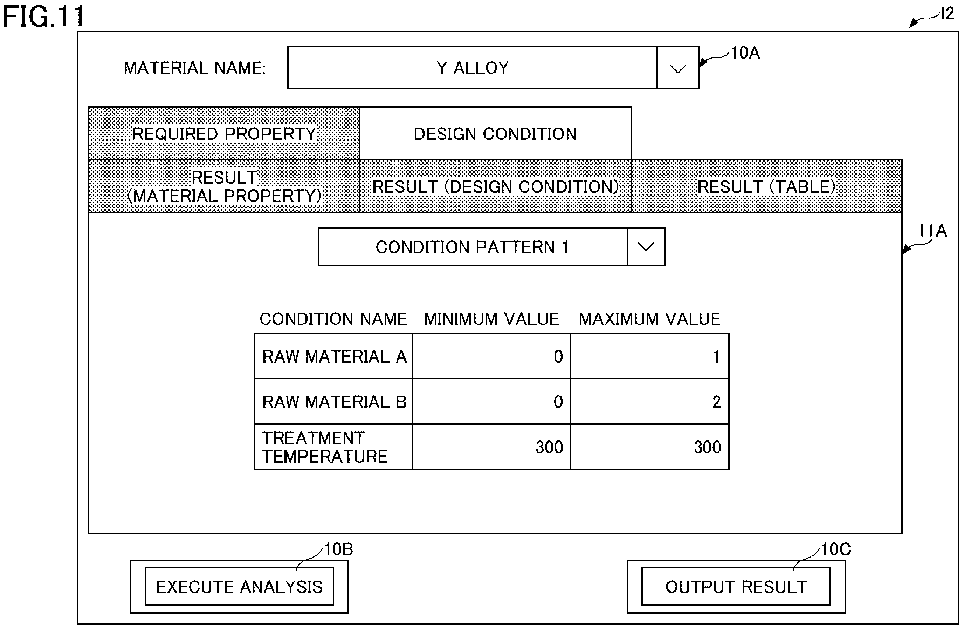

FIG. 11 is a diagram illustrating an example of an input screen 11A of the design condition setting unit 11 in the material design interface 12. In the material design interface 12, the input screen 11A is displayed as a tabbed window of "design conditions." In the material design interface 12, a pull-down list 10A for selecting a material name to be designed is displayed, and in FIG. 11, "Y alloy" is selected as the material to be designed. In this case, for example, the design condition setting unit 11 can read the names file described with reference to FIG. 9 and create the pull-down list 10A of selectable material names. Further, the design condition setting unit 11 reads the learned model associated with the selected material name.

The design conditions include items related to the composition of raw materials ("raw material A" and "raw material B" in FIG. 11) and items related to production conditions ("treatment temperature" in FIG. 11). On the input screen 11A, a condition pattern can be selected from a pull-down menu. In this case, for example, the design condition setting unit 11 reads the names file described with reference to FIG. 9 and creates the pull-down menu of selectable condition patterns. The items that can be selected as the condition pattern are determined by being associated with the material name.

For example, in the case where the material to be designed is an aluminum (Al) alloy, the composition of the raw material includes elements such as Si, Fe, Cu, Mn, Mg, Cr, Ni, Zn, Ti, Na, V, Pb, Sn, B, Bi, Zr, O, and the like as an additive in percentage by mass (wt%). Note that the percentage by mass of Al is represented by 100%- (the sum of the percentage by mass of the above-described elements).

For example, if the material to be designed is an aluminum alloy, as the items of the production condition, the items related to a heat treatment include, for example, the temperature (°C) and the execution time (h) of each processing, such as annealing, a solution heat treatment, an artificial aging treatment, a natural aging treatment, a hot working treatment, a cold working treatment, and a stabilizing treatment. The items related to processing conditions include, for example, a processing rate, an extrusion rate, a reduction of area, and a product shape.

After the condition pattern is selected in the input screen 11A and the design conditions are set, the "execute analysis" button 10B of the material design interface I2 is pressed to start the processing of the forward problem analysis unit 10.

The comprehensive prediction point generation unit 12 generates multiple comprehensive prediction points within the specified range of the design conditions set by the design condition setting unit 11. For example, in a case where a percentage by mass of Si in the composition item and a range of annealing execution time in the production condition item are specified, first, a plurality of numerical values are calculated within a specified range of the percentage by mass of Si and within the specified range of the annealing execution time in random or predetermined intervals, and all combinations of the plurality of numerical values in each item are generated. These combinations are output as comprehensive prediction points.

The learned model 13 is a model formulated by acquiring the correspondence between the input information including the design condition of the aluminum alloy and the output information including the material property value acquired by machine learning. As for the learned model 13, in the example of FIG. 11, a learned model used when the material to be designed is "Y alloy" is created by the expert terminal 2, and is available by the general-purpose terminal 3.

The items of material properties include tensile strength, 0.2% strength, elongation, a linear expansion coefficient, Young's modulus, a Poisson's ratio, a fatigue property, hardness, and creep properties including creep strength and creep strain, shear strength, specific heat capacity, thermal conductivity, electrical resistivity, density, a solidus line, a liquidus line and the like.

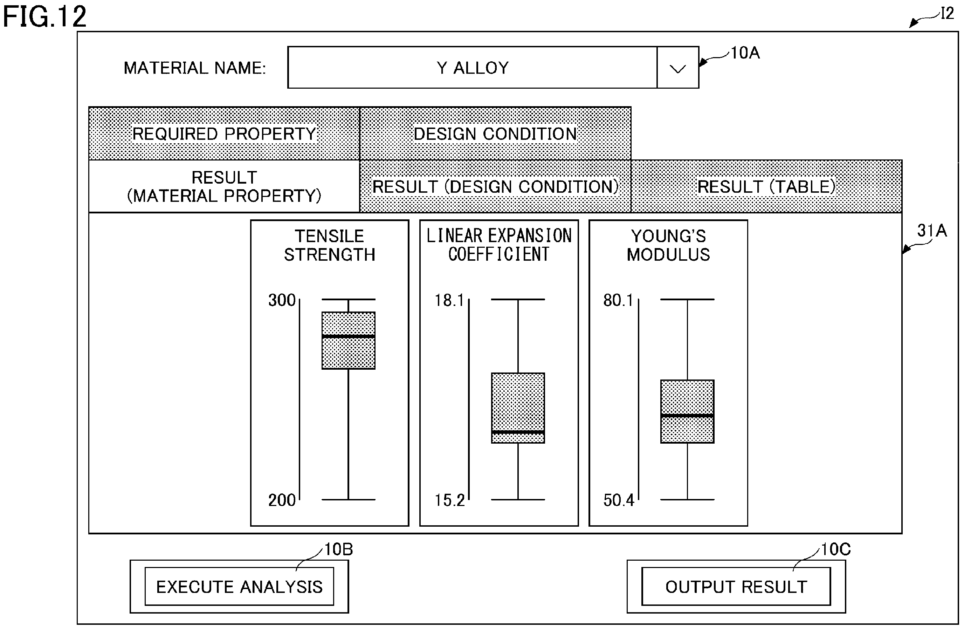

FIG. 12 is a diagram illustrating an example of an output screen 31A of the forward problem analysis unit 10 in the material design interface 12. In the material design interface 12, the output screen 31A is displayed as a tabbed window of "results (material properties)." The output screen 31A is displayed on the material design interface 12, for example, through an information display unit 31. In FIG. 12, the output (material properties) of the learned model 13 is limited to only three, i.e., "tensile strength", "linear expansion coefficient", and " Young's modulus", for simplicity of explanation, but it is not limited to this. In the example of FIG. 12, the range of values for each material property is represented by box plots.

The design condition-material property table 14 stores data sets in which the material property values calculated by inputting the comprehensive prediction points generated by the comprehensive prediction point generation unit 12 into the learned model 13 are associated with the respective points of the comprehensive prediction points. When performing the calculation of the comprehensive prediction points by the learned model 13, the forward problem analysis unit 10 stores the output in the design condition-material property table 14 by associating with the comprehensive prediction points (inputs). In the design condition-material property table 14, the inputs (production conditions, material compositions) and the output (material properties) of a learned model are put together as one data set and recorded on the same row of the design condition-material property table 14. Each row of the design condition-material property table 14 is an individual data set, and each column records numerical values of each item of the inputs and the output of the learned models 13. The design condition-material property table 14 is stored in association with the material name selected in the input screen 11A of FIG. 11.

As described above, the forward problem analysis unit 10 is configured to automatically generate data sets of design conditions and material properties covering the entire range of multidimensional design conditions in response to the material designer simply performing the operations of specifying the range of the multidimensional design conditions.

The reverse problem analysis unit 20 is provided with a required property setting unit 21 and a design condition extraction unit 22. Further, the above-described design condition-material property table 14 is also included in the reverse problem analysis unit 20.

The required property setting unit 21 sets a specified range of a required property of the material to be designed. The required property setting unit 21 can set specified ranges of required properties by, for example, displaying an input screen for required properties on the material design interface I2 to prompt the material designer to input specified ranges.

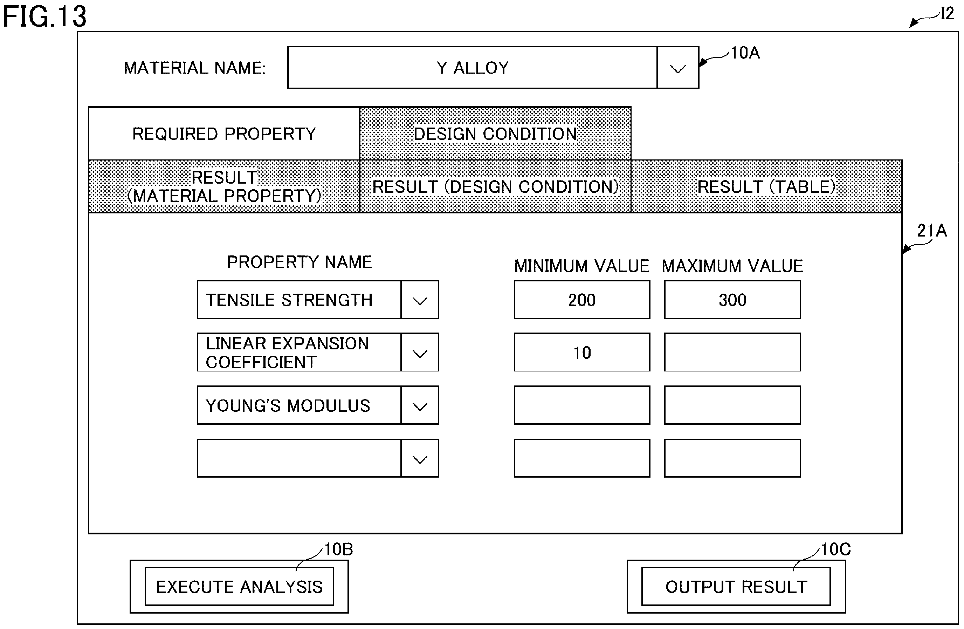

FIG. 13 is a diagram illustrating an example of an input screen 21A of the required property setting unit 21. In the material design interface 12, the input screen 21A is displayed as a tabbed window of "required properties." In the material design interface 12, the pull-down list 10A for selecting the name of the material to be designed is displayed, and in FIG. 13, "Y alloy" is selected as the material to be designed. In this case, for example, the required property setting unit 21 can read the names file described with reference to FIG. 9 and create the pull-down list 10A of the selectable material names. The required property setting unit 21 reads the design conditions associated with the selected design condition-material property table 14.

The items of required properties are the same as those of the material properties described above. In the input screen 21A, a property name can be selected using the pull-down menu. In this case, for example, the design condition setting unit 11 reads the names file described with reference to FIG. 9 to generate a pull-down menu of the selectable property names. The items that can be selected as property names are determined by associating the items to the material names. In the example of FIG. 13, "tensile strength," "line expansion coefficient," and "Young's modulus" are selected as the required properties. In the input screen 21A, the maximum value and the minimum value of each property can be input.

The processing of the reverse problem analysis unit 20 is started after selecting a property name in the input screen 21A and setting the required property, and then by clicking the "execute analysis" button 10B of the material design interface 12.

The design condition extraction unit 22 extracts a data set that satisfies the required properties set by the required property setting unit 21 from the design condition-material property table 14.

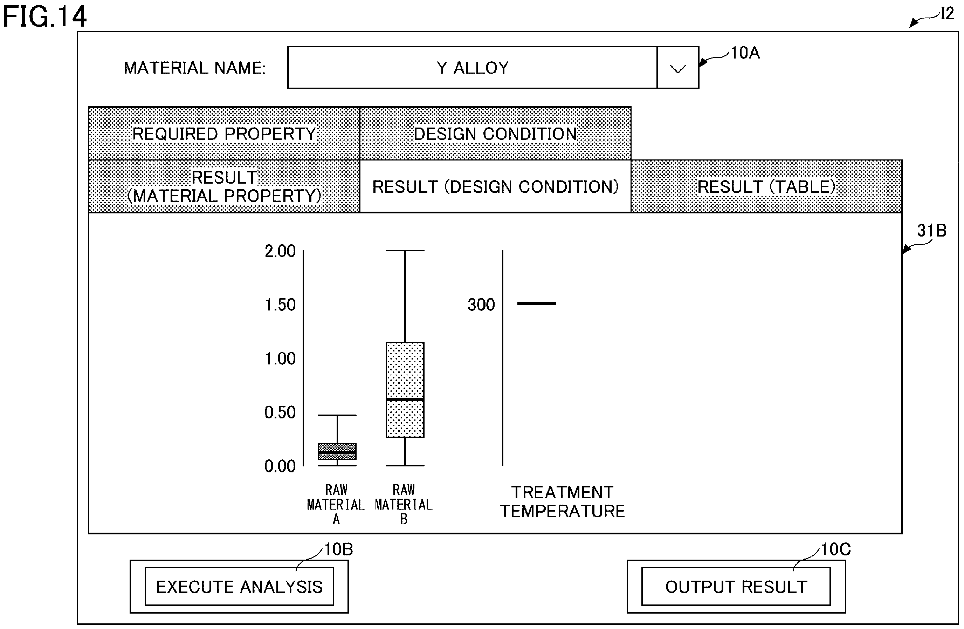

FIG. 14 is a diagram illustrating an example of an output screen 31B of the reverse problem analysis unit 20. In the material design interface 12, the output screen 31B is displayed as a tabbed window of "results (design condition)." The output screen 31B is displayed on the material design interface 12, for example, through the information display unit 31. In the output screen 31B illustrated in FIG. 14, the range of the composition (raw material A, raw material B) and the production conditions (treatment temperature) satisfying all the required properties set in FIG. 13 are represented by box plots. At this time, when the tabbed window of the above-described "results (material property)" is switched, the range of values of each material property is displayed by updating the range of values of each material property corresponding to the design condition illustrated in FIG. 14.

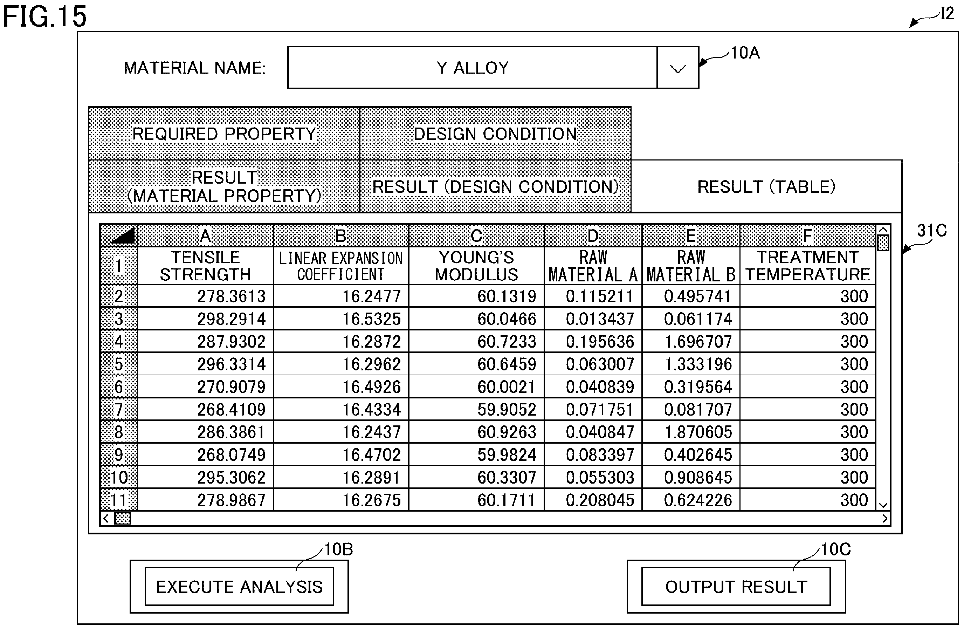

FIG. 15 is a diagram illustrating an output screen 31C which is another example of an output screen of the reverse problem analysis unit 20. In the material design interface 12, the output screen 31C is displayed as a tabbed window of "results (table)." The output screen 31C is displayed on the material design interface 12, for example, through the information display unit 31. The output screen 31C illustrated in FIG. 15 displays a table in which the required properties (tensile strength, linear expansion coefficient, and Young's coefficient) set in FIG. 13 and design conditions (raw material A, raw material B, and treatment temperature) satisfying all of the required properties illustrated in FIG. 14 are summarized. Further, the table may be in a mode in which the display portion of the table can be moved by a scroll bar.

Further, in the output screen 31C, the "output result" button 10C of the material design interface I2 can be pressed to output the table illustrated in FIG. 15 to the outside in a format such as a CSV file.

As described above, the reverse problem analysis unit 20 is configured such that the production conditions (material compositions or design conditions) satisfying multidimensional required properties can be simultaneously extracted in response to the material designer simply performing the operations of specifying the range of the multidimensional required properties conditions. Further, without using a simulation or a learning model for the reverse problem analysis, the design condition-material property table 14 generated by the forward problem analysis unit 10 is used. Therefore, the calculation cost can also be significantly reduced.

The input/output unit 30 includes the information display unit 31.

The information display unit 31 displays the output of the forward problem analysis unit 10 or the output of the reverse problem analysis unit 20. For example, as illustrated in FIG. 14 and FIG. 15, the range of the required property and the range of the design condition relating to the data set extracted by the design condition extraction unit 22 are displayed.

The input/output unit 30 may further include a design condition adjustment unit 32.

The design condition adjustment unit 32 adjusts the range of the design condition of the data set extracted by the design condition extraction unit 22. The design condition adjustment unit 32 can adjust the range of the design condition by, for example, the material designer's input operation of changing the composition range of the output screen 31B to be displayed on the material design interface 12.

Further, the design condition extraction unit 22 can further narrow down the data sets extracted according to the required properties to data sets satisfying the design condition adjusted by the above-described design condition adjustment unit 32.

The reverse problem analysis unit 20 outputs the design conditions satisfying the required properties, but these design conditions are only those automatically extracted from the comprehensive prediction points of the design condition-material property table 14, and production constraints, such as a difficulty of the actual production, have not been considered. For example, there are various production constraints, such as a difficulty of producing due to the difficulty of handling, production taking a long time, processing taking a long time, a composition with pores caused during casting, impossible to mold, possible to produce without considering the cost but impossible to produce by using an ordinary plant facility. In a case where the input/output unit 30 includes the design condition adjustment unit 32, it is possible to narrow down the production conditions satisfying the required properties considering the production constraints based on the material designer's practical experience by allowing the material designer to adjust the output results of the reverse problem analysis unit 20 with the design condition adjustment unit 32. That is, it becomes possible to perform the material design in which the prediction by machine learning and the material designer's experience work together.

As described above, in the present embodiment, during the performance of the forward problem analysis, data sets to be used in the reverse problem analysis are generated and stored in the design condition-material property table 14. At the time of performing the reverse problem analysis, data sets satisfying the required properties are extracted by referring to the design condition-material property table 14. In other words, the reverse problem analysis performs only the task of searching for the design condition-material property table 14 without performing any numerical value calculations, such as simulation and model calculation. Therefore, the calculation cost can be greatly reduced, and the optimal solution of the design condition satisfying the desired material properties can be derived in a short time.

Further, in a case of performing a reverse problem analysis by a conventional simulation or in a case of adopting a machine learning system to reverse problem analysis, when where there is a plurality of required properties, calculations are performed to gradually reach the optimal solution while performing the adjustment for each property in turn, and candidate material searches will not be collectively performed to satisfy several types of properties at the same time. In many cases, a plurality of material properties has a trade-off relationship, and an optimal solution is reached through repeated trial and error. Therefore, it takes a long time to acquire the optimal solution of the design conditions satisfying the desired material properties. In contrast to this, in the present embodiment, by setting a plurality of output (material properties) of the learned model 13 and generating items of a plurality of material properties in the design condition-material property table 14, in the reverse problem analysis, the candidate material searches can be collectively performed to satisfy the plurality of types of material properties. This allows, even in the case of setting a plurality of types of required properties, the time required to derive the optimal solution to be greatly reduced as compared with the conventional method.

Further, the data set group stored in the design condition-material property table 14 is information derived from a large number of comprehensive prediction points automatically generated in the forward problem analysis. Therefore, the increment of each item of the design condition and the material property is sufficiently small, and the resolution is high. Therefore, in the reverse problem analysis, the prediction of the design condition satisfying the required property can be performed with high accuracy.

Preferably, the general-purpose terminal 3 is provided with the design condition adjustment unit 32 for adjusting the range of the design condition of the data set extracted by the design condition extraction unit 22. In a case where the general-purpose terminal 3 includes the design condition adjustment unit 32, the design condition extraction unit 22 further narrows down the data sets satisfying the design conditions adjusted by the design condition adjustment unit 32.

In this case, depending on the required properties, the design condition extraction unit 22 can perform the narrowing down of the design condition automatically extracted by the design condition-material property table 14 by considering the production constraints and the like based on the experience of the material designer. This enables to perform the material design in which the prediction by machine learning and the material designer's experiences work together, which in turn can extract design conditions that are easier to perform the production.

Further, the general-purpose terminal 3 of the present embodiment is provided with the information display unit 31 for displaying the required properties for the data sets extracted by the design condition extraction unit 22 and the range of the design condition. Further, in a case where the general-purpose terminal 3 is provided with the design condition adjustment unit 32, the design condition adjustment unit 32 adjusts the range of the design condition according to the user's operation of changing the range of the design condition displayed on the information display unit 31.

In a case where the general-purpose terminal 3 is provided with the design condition adjustment unit 32, the adjustment operation of the range of the design condition by the material designer can be performed more intuitively on the input/output unit 30, which can be simplified by reducing the burden of the adjustment operation. Further, the result of the adjustment operation can be reflected immediately. Therefore, the interactive adjustment operation by the material designer can be performed, which makes it possible to perform the adjustment of the range of the design condition more efficiently.

Further, as described with reference to FIG. 11 to FIG. 15, the material design interface I2 available in the general-purpose terminal 3 allows the design of the selected material by selecting the material to be designed in the pull-down list 10A for selecting the name of the material to be designed. Therefore, the interface can be shared in the design works of a plurality of materials without preparing individual interfaces for each material, and the versatility can be improved. Generally, materials manufacturers have developed a wide variety of materials in a number of departments, so it is very useful to provide a highly versatile interface that can be deployed throughout the organization.

### <Hardware configuration of each terminal>

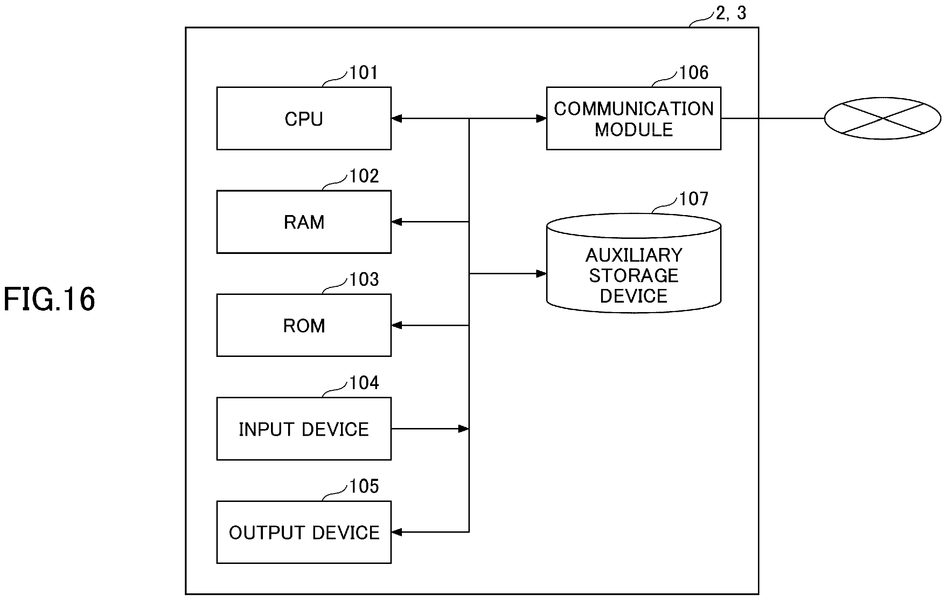

FIG. 16 is a diagram illustrating a hardware configuration of the expert terminal 2 and the general-purpose terminal 3 as blocks. As illustrated in FIG. 16, the expert terminal 2 and the general-purpose terminal 3 may be configured as a computer system physically including physically a Central Processing Unit (CPU) 101, a Random Access Memory (RAM) 102 as a main storage device, a Read Only Memory (ROM) 103, an input device 104 such as a keyboard and a mouse, an output device 105 such as a display, a communication module 106 which is a data transmission/reception device such as a network card, and an auxiliary storage device 107 such as a hard disk.

Each function of the expert terminal 2 illustrated in FIG. 3 and the functions of the general-purpose terminal 3 illustrated in FIG. 10 is implemented by reading predetermined computer software (material design program) on hardware, such as a CPU 101 and a RAM 102 to operate the communication module 106, the input device 104, and the output device 105 under the control of the CPU 101 and to read and write the data in the RAM 102 and the auxiliary storage device 107. That is, by running the material design program of the present embodiment on a computer, the material design system 1 functions as the learning condition setting unit 41, the model learning unit 42, and the model transmission unit 43 of FIG.3, and the design condition setting unit 11, the comprehensive prediction point generation unit 12, the learned model 13, the required property setting unit 21, the design condition extraction unit 22, the information display unit 31, and the design condition adjustment unit 32 of FIG. 10, respectively. Further, it is possible to implement a learning function of performing machine learning of a model that inputs/outputs the correspondence between the design conditions and the material property value of the material to be designed. Further, it is possible to implement an estimation function of estimating the material property value from the design condition or estimating the design condition from the material property value, with respect to the specific material to be designed, using the learned model 13 for the specific material to be designed created by the learning function. Further, it is also possible to implement a data set generation function of storing a data set in which the material property value calculated by inputting the comprehensive prediction point generated by the comprehensive prediction point generation function to the learned model 13 is associated with each point of the comprehensive prediction points to the design condition-material property table 14. The design condition-material property table 14 illustrated in FIG. 10 can be implemented by a part of a storage device (the RAM 102, the ROM 103, the auxiliary storage device 107, or the like) provided in the computer. Further, the model learning interface I1 and the material design interface I2 illustrated in FIG. 1 and FIG. 2, and the input/output unit 30 illustrated in FIG. 10 can be implemented by the output device 105 or the input device 104 provided in a computer.

The material design program of the present embodiment is stored, for example, in a storage device provided by a computer. The material design program may be configured such that a part or all of the program is transmitted via a transmission medium, such as a communication line, and is received and recorded (including "installation") by a communication module or the like provided in a computer. The material design program may also be configured such that a part or all of the program may be recorded (including "installation") in a computer from a state in which the program is stored in a portable storage medium, such as a CD-ROM, a DVD-ROM, and flash memory.

### <Material design method>

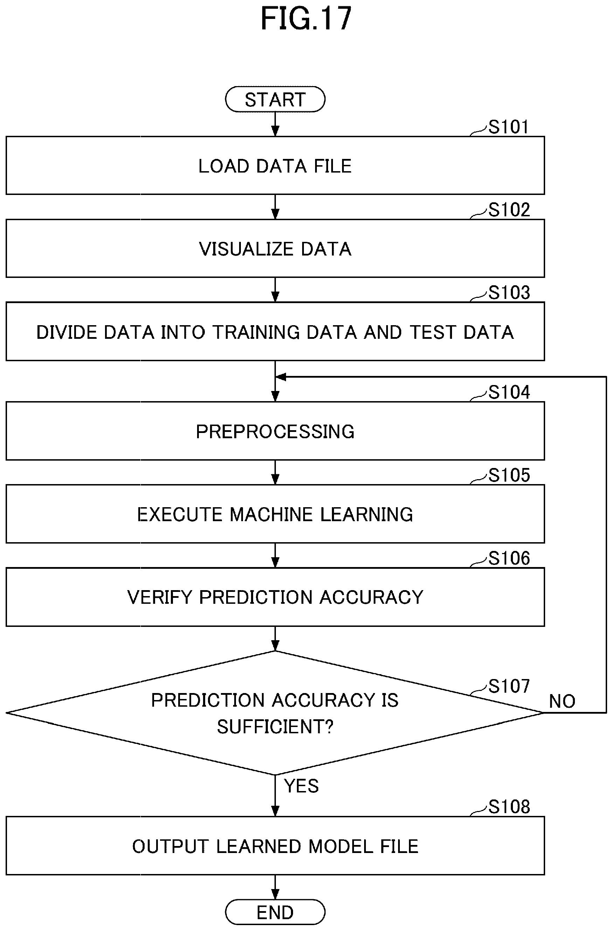

A material design method by the material design system 1 according to the present embodiment will be described with reference to FIG. 17 to FIG. 19.

First, a model learning processing performed by the expert terminal 2 will be described with reference to FIG. 17. FIG. 17 is a flowchart of the model learning processing performed by the expert terminal 2.

In step S101, a data file for model learning is read out by the learning condition setting unit 41. For example, as illustrated in the input screen 41A of FIG. 4, a data file is selected and read according to an operator's selection operation using the file selection box 40A of the model learning interface I1.

In step S102, the learning condition setting unit 41 visualizes the read data. For example, as illustrated in the input screen 41A of FIG. 4, the data set included in the read data file is displayed in a table, and the numerical values included in the data set are illustrated in a histogram, a scatter plot, and the like.

In step S103, the learning condition setting unit 41 divides the data set included in the read data file into training data and test data. For example, as illustrated in the input screen 41B of FIG. 5, data is divided based on various conditions set by the operator.

In step S104, preprocessing of the data set for model learning is performed by the model learning unit 42. For example, as illustrated in the input screen 41C of FIG. 6, a variable transformation, normalization, and the like is performed based on various conditions related to the preprocessing set by the operator.

In step S105, the model learning unit 42 executes the machine learning of the model. For example, as illustrated in the input screen 41D of FIG. 7, machine learning of the model is performed based on various conditions related to machine learning set by an operator.

In step S106, the model learning unit 42 verifies the prediction accuracy. For example, as illustrated in the input screen 41E of FIG. 8, the verification process is performed based on various conditions of the accuracy verification set by the operator.

In step S107, whether the prediction accuracy is sufficient or not is determined by the model learning unit 42. For example, as illustrated in the input screen 41E of FIG. 8, when the condition of the verification result set by the operator is satisfied, the prediction accuracy can be determined to be sufficient. When the prediction accuracy is insufficient (NO in step S107), the machine learning of the model is repeated by returning to step S104. If the prediction accuracy is sufficient (YES in step S107), the process proceeds to step S108.

In step S108, the learned model file is output by the model transmission unit 43. The model transmission unit 43 outputs the learned model and the names file including various names (material name, material property name, and design condition item name) set in FIG. 9. When the process in step S108 is completed, the control flow ends.

A series of processing by the expert terminal 2 of the flowchart illustrated in FIG. 17 corresponds to a "learning step for machine learning of a model in which the correspondence between the design condition and the material property value of the material to be designed is determined as the input/output" in the material design method according to the present embodiment.

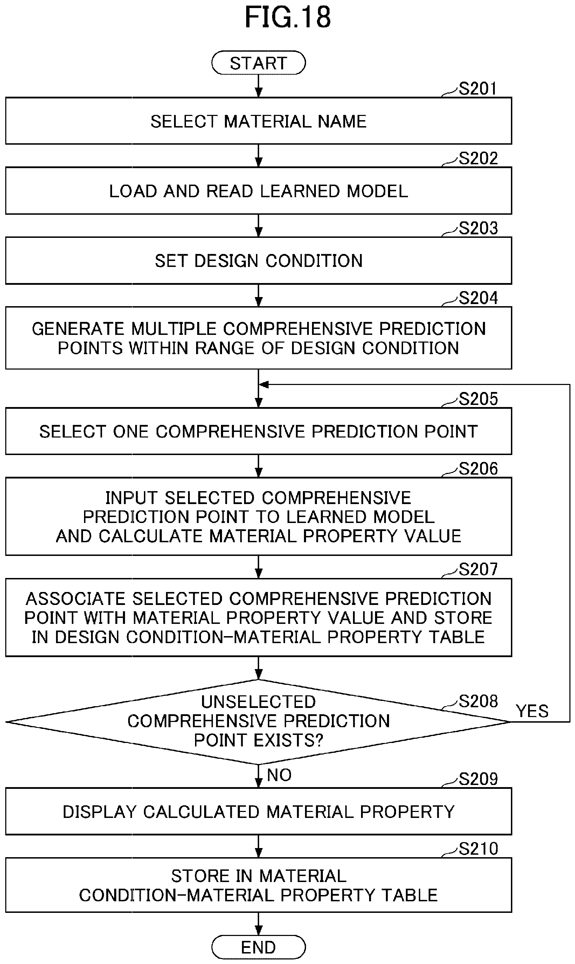

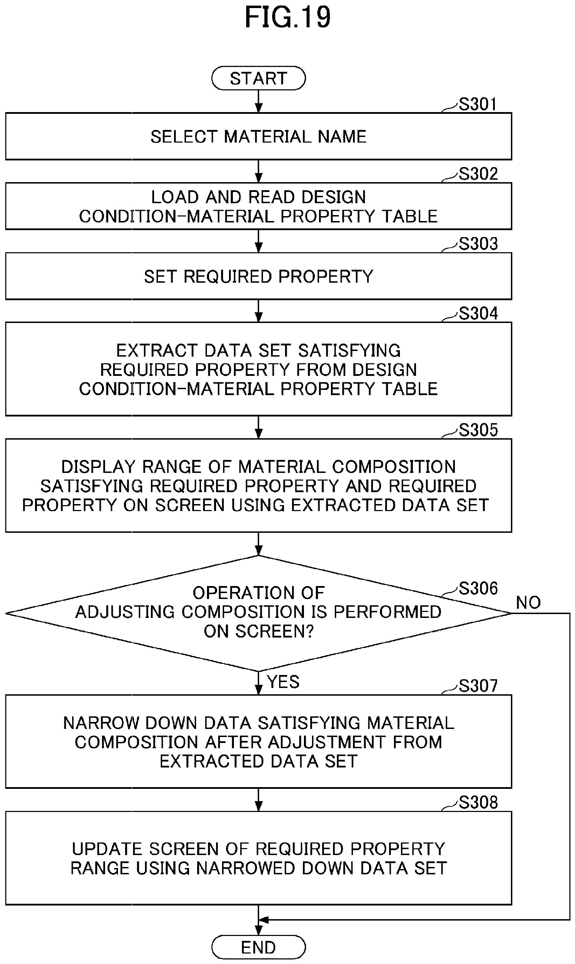

Next, a material design processing performed by the general-purpose terminal 3 will be described with reference to FIG. 18 and FIG. 19. Fig. 18 is a flowchart of the forward problem analysis processing performed by the forward problem analysis unit 10 of the general-purpose terminal 3.

Before performing the forward problem analysis processing of FIG. 10, the learned model 13, which is obtained by the correspondence between the input information including the design condition and the output information including the material property value of the material to be designed, is used for the material design interface I2 of the general-purpose terminal 3.

In step S201, the material name of the material to be designed is selected by the design condition setting unit 11. For example, as illustrated in the input screen 11A of FIG. 11, the material name is selected according to the operator's selection operation through the pull-down list 10A of the material design interface 12.

In step S202, the design condition setting unit 11 loads and reads the learned model associated with the material name selected in step S201.

In step S203, the design condition setting unit 11 sets the range of the design condition of the material to be designed (design condition setting step). For example, the design condition setting unit 11 displays the input screen 11A illustrated in FIG. 11 on the material design interface I2 to prompt the material designer to input the specified range.

In step S204, multiple comprehensive prediction points are generated by the comprehensive prediction point generation unit 12 (comprehensive prediction point generation step) within a specified range of the design condition set in step S203.

In steps S205 to S208, the forward problem analysis unit 10 stores the material property value generated in step S204 by inputting the comprehensive prediction point generated in step S204 into the learned model 13 in the design condition-material property table 14 in which the data set is associated with each point of the comprehensive prediction point (data set creation step) .

First, one comprehensive prediction point is selected in step S205, and the selected comprehensive prediction point in step S205 is input to the learned model 13 to calculate the material property value in step S206. Then, in step S207, the prediction point of the input of the learned model 13 selected in step S205 and the material property value of the output are associated and stored in the design condition-material property table 14. Through the processing of steps S205 to S207, one data set is generated.

In step S208, whether unselected comprehensive prediction point exists is determined. If an unselected comprehensive prediction point exists (YES in step S208), return to step S205 to repeat the data set generation.
If all of the comprehensive prediction points have been selected (NO in step S208), the data set generation is completed, and the process proceeds to step S209.

In step S209, the material properties of each prediction point calculated in step S206 are displayed on the material design interface I2 through the information display unit 31. The information display unit 31 displays the output screen 31A illustrated in FIG. 12 on the material design interface 12.

In step S210, the forward problem analysis unit 10 generates a data set in which the material property value calculated by the input of the comprehensive prediction point generated by the comprehensive prediction point generation unit 12 into the learned model 13 is associated with each point of the comprehensive prediction points, and stores the data set in the design condition-material property table 14. The design condition-material property table 14 is stored in association with the material name selected in the input screen 11A of FIG. 11. When the process of step S210 is completed, the forward problem analysis processing of the control flow is completed.

FIG. 19 is a flowchart of the reverse problem analysis process performed by the reverse problem analysis unit 20 and the design condition adjustment unit 32 of the general-purpose terminal 3.

In step S301, the material name of the material to be designed is selected by the required property setting unit 21. For example, as illustrated in the input screen 21A of FIG. 13, the material name is selected according to the operator's selection operation through the pull-down list 10A of the material design interface 12.

In step S302, the required property setting unit 21 loads and reads the design condition-material property table 14 associated with the material name selected in step S301.

In step S303, a range of the required property of the material to be designed is set by the required property setting unit 21 (required property setting step). For example, the required property setting unit 21 displays the input screen 21A illustrated in FIG. 13 on the material design interface I2 to prompt the material designer to input the specified range.

In step S304, the design condition extraction unit 22 extracts the data set satisfying the required property set in step S303 from the design condition-material property table 14 (design condition extraction step).

In step S305, the information display unit 31 displays the range of the material composition satisfying the required property specified in step S303 and the required property on the material design interface I2 using the data set extracted in step 304. The information display unit 31 displays the output screen 31B illustrated in FIG. 14 on the material design interface 12.

In step S306, the design condition adjustment unit 32 determines whether an operation of the composition adjustment by the material designer is performed on the output screen 31B indicating the range of the material composition satisfying the required property. The material designer can perform an operation to change the position of the maximum and minimum values in the box plots of the material composition of the output screen 31B (design condition adjustment step). If this operation is performed (YES in step S306), the design condition adjustment unit 32 outputs information of the composition range after adjustment by the design condition extraction unit 22 and proceeds to step S307. If no operation is performed (NO in Step S306), the reverse problem analysis process of the present control flow is completed.

In step S307, since the composition adjustment operation is detected in step S306, the design condition extraction unit 22 narrows down the data satisfying the material composition after the adjustment of the composition range from among the data set groups extracted in step S304 (narrowing down step).

In step S308, the information display unit 31 updates the output screen 31B of the required property displayed in step S305 using the data set narrowed down in step 307. When the process in step S308 is completed, the reverse problem analysis process is completed.

A series of processes performed by the general-purpose terminal 3 of the flowchart illustrated in FIG. 18 and FIG. 19 corresponds to "an estimation step of estimating a material property value from the design condition or an estimation step of estimating a design condition from the material property value using the learned model 13 for the specified material to be designed created in the learning step for the specified object material to be designed" in the material design method according to the present embodiment.

As described above, the embodiment has been described with reference to specific examples. However, the present disclosure is not limited to these specific examples. Modifications in which these specific examples are appropriately modified by those skilled in the art are also encompassed by the scope of the present disclosure as long as they are provided with the features of the present disclosure. Each element included in each of the specific examples described above and the arrangement, condition, shape, and the like thereof are not limited to those exemplified and can be changed as appropriate. Each element provided in each of the above-described specific examples can be appropriately changed in the combination as long as no technical inconsistency occurs.

In the above-described embodiment, the configuration of the general-purpose terminal 3 including the forward problem analysis unit 10 and the reverse problem analysis unit 20 is illustrated. However, the general-purpose terminal 3 may be configured to analyze only one of the forward problem or the reverse problem. In the configuration in which the general-purpose terminal 3 performs only the reverse problem analysis, the relationship between the input and output of the model is changed from the above-described embodiment. The model input is the material property, and the model output is the design condition, and the reverse problem analysis is performed using the learned model of the input/output relationship. That is, the general-purpose terminal 3 may be configured to perform processing using the learned model 13 created by the expert terminal 2.

This international application claims priority under Japanese Patent Application No. 2019-135216, filed on July 23, 2019, and the entire contents of Japanese Patent Application No. 2019-135216 are incorporated herein by reference.

### [Reference Signs List]

- 1: material design system

- 2: expert terminal
- 3: general-purpose terminal
- 4: intermediate device
- 11: design condition setting unit
- 12: comprehensive prediction point generation unit
- 13: learned model
- 14: design condition-material property table
- 21: required property setting unit
- 22: design condition extraction unit
- 31: information display unit
- 32: design condition adjustment unit
- 41: learning condition setting unit
- 42: model learning unit
- 43: model transmission unit
- I1: model learning interface
- I2: material design interface

## Claims

1. A material design system for designing a material to be designed including a material composed of a plurality of compositions or a material produced by combining a plurality of production conditions, the material design system comprising:
an expert terminal configured to use a model learning interface for performing machine learning of a model that inputs and outputs a correspondence between a design condition and a material property value of the material to be designed; and
a plurality of general-purpose terminals configured to use a material design interface for estimating the material property value based on the design condition or estimating the design condition based on the material property value, by using a learned model that is created by the expert terminal and is for the material to be designed.

2. The material design system according to claim 1, further comprising an intermediate device for storing the learned model created by the expert terminal,
wherein the plurality of general-purpose terminals, by using the learned model stored in the intermediate device, estimate the material property value based on the design condition or estimate the design condition based on the material property value.

3. The material design system according to claim 1 or 2, wherein communication between the model learning interface and the material design interface is performed via a network line.

4. The material design system according to claim 1 or 2, wherein the model learning interface and the material design interface are installed in a cloud server, and communication between the model learning interface and the material design interface is performed by communication in the cloud server.

5. The material design system according to claim 1 or 2, wherein the model learning interface and the material design interface are included in separate software compatible with each other.

6. The material design system according to any one of claims 1 to 5, wherein the expert terminal includes:
a learning condition setting unit configured to set various conditions for machine learning of the model;
a model learning unit configured to perform machine learning of the model based on the various conditions; and
a model output unit configured to output the learned model.

7. The material design system according to any one of claims 1 to 6, wherein the general-purpose terminal includes:
a design condition setting unit configured to set a specified range of the design condition of the material to be designed;
a comprehensive prediction point generation unit configured to generate a plurality of comprehensive prediction points within the specified range set by the design condition setting unit;
a design condition-material property table for storing a data set associated with each point of the comprehensive prediction point, wherein the material property value is calculated by inputting the comprehensive prediction point generated by the comprehensive prediction point generation unit into the learned model;
a required property setting unit configured to set a specified range of a required property of the material to be designed; and
a design condition extraction unit configured to extract a data set satisfying the required property set by the required property setting unit from the design condition-material property table.

8. The material design system according to claim 7, wherein the general-purpose terminal further includes a design condition adjustment unit configured to adjust a range of the design condition of the data set extracted by the design condition extracting unit, and
the design condition extraction unit further narrows down the data set satisfying the design condition adjusted by the design condition adjustment unit from the extracted data set.

9. A material design method of designing a material to be designed including a material composed of a plurality of compositions or a material produced by combining a plurality of production conditions, the material design method comprising:
performing machine learning of a model that inputs and outputs a correspondence between a design condition and a material property value of the material to be designed; and
estimating the material property value based on the design condition or estimating the design condition based on the material property value, by using a learned model that is created by the expert terminal and is for the material to be designed.

10. A material design program for designing a material to be designed including a material composed of a plurality of compositions or a material produced by combining a plurality of production conditions, the material design program causing a computer to implement as:
a learning function of performing machine learning of a model that inputs and outputs a correspondence between a design condition and a material property value of the material to be designed; and
an estimation function of estimating the material property value from the design condition or estimating the design condition from the material property value, by using a learned model that is created by the expert terminal and is for the material to be designed.
